# EUROPEAN PATENT SPECIFICATION

(11) **EP 1 743 603 B1**
(45) Date of publication and mention of the grant of the patent: **11.12.2013**
(21) Application number: 06253579.4
(22) Date of filing: 07.07.2006
(51) Int. Cl.: A61F 2/915

(54) **Flexible stent with excellent expandability and trackability**
Flexibler Stent mit bester Dehnbarkeit und Verfolgbarkeit
Stent flexible à l'expansibilité et au guidage excellents

(30) Priority: 11.07.2005 JP 2005201406
(43) Date of publication of application: 17.01.2007
(73) Proprietor: NIPRO CORPORATION, Osaka-shi, Osaka 531-8510 (JP)
(72) Inventor: Sano, Yoshihiko, Osaka 531-8510 (JP)
(74) Representative: Setna, Rohan P.

(56) References cited:
- WO-A-00/15151
- WO-A-01/26584
- WO-A-99/49810

## Description

### Technical Field

The present invention relates to a stent to be implanted in a living body to maintain a luminal diameter of a body cavity such as the blood vessel.

### Background Art

Stents have been used to expand luminal diameters of body cavities such as blood vessels and keep the resultant luminal sizes of the body cavities. There are various methods for expanding such a stent, including balloon dilation, self-expansion using a shape memory material, mechanical expansion or the like. Among them, the most widely used method is the balloon dilation. In the balloon dilation, a stent is introduced into a desired site in the body together with a balloon catheter and expanded by inflation of the balloon to dilate a luminal diameter of the body cavity. The stent generally comprises luminal diameter-holding portions for dilating and holding the luminal diameter of the body cavity such as the blood vessel, and joint portions for connecting the luminal diameter-holding portions in the longitudinal direction of the stent. After being expanded, the stent maintains its expanded shape.

Many stents comprising luminal diameter-holding portions and joint portions are being proposed. Included in such proposed stents are, for example, a stent comprising plural cylindrical components which are separately expandable in the radial direction thereof and are connected with one another so that they are substantially aligned along the common axis(Patent'Document 1); a stent comprising a tubular member expandable in the radial direction, the tubular member being constituted by a plurality of elongated members intersecting with one another (Patent Document 2); a stent comprising at least two unitary wire-like circular members each bent to form a plurality of substantially straight, non-overlapping segments connected at axial bends; the at least two circular members having at least one pair of aligned axial bends; and the at least two circular members connected by at least one substantially rigid joint at least one pair of aligned axial bends (Patent Document 3); a stent comprising a tube having a patterned shape which has first and second meander patterns having axes extending in first and second directions (Patent Document 4); and a stent of an open structure comprising plural cylindrical segments defined by interconnected struts, the segments being interconnected at end portions thereof by a plurality of diagonal interconnecting elements (Patent Document 5).
Patent Document 1: JP-H06-181993 A
Patent Document 2: JP S62-231657 A
Patent Document 3: JP H08-155035 A
Patent Document 4: JP H10-503676 A
Patent Document 5: JP H11-505441 A

These stents of the prior art have been improved; but they may cause obstruction or stenosis of the lumen since the stent, when being expanded, still put a load on the lumen such as the blood vessel in the vicinity of edges of the stent. Since these stents have still-inadequate flexibility, it is often difficult to introduce the stent into an objective site when the lumens are of a three-dimensional meandering structure. In addition, Lhe stents may cause injury to the blood vessel during introduction of the stent into the objective site. When the blood vessel has a branched blood vessel at the site of stent placement, it is frequently difficult to provide the placed stent with a lateral hole. When being expanded, these stents may cause so-called shortening, i.e., they are shortened in length. WO 00/15151 relates to a linkage stent.

### Disclosure of Invention

In view of the above circumstances, the present invention has been made to provide a flexible stent with excellent expandability and trackability, which makes it possible to pass through three-dimensional meandering lumens, has a good radial strength and makes it possible to provide a lateral hole.

According to the present invention, there is provided a flexible stent according to claim 1 of the claims appended hereto.

In the present invention, the two waved elements have such a basic pattern that they are in parallel with each other. However, the two waved elements may have such a modified pattern that they are substantially parallel to each other, i.e., a gap between two waved elements is uneven and is narrowed or widened the tops of the wave crests and the bottoms of the wave troughs of the two waved elements in pair. Further, the gap between two waved elements is set within the range of 40 to 70 µm in view of accuracy of current laser beam machining. This results from the following reasons. Firstly, it is difficult to reduce the gap between the two waved elements to less than 40 µm because of present technical problems. Secondary, the distance greater than 70 µm causes a problem in radial strength of the stent as the number of meanderings of the waved elements is decreased. The number of the coupling element that couples the two waved elements is not limited to one, and the two waved elements may be coupled by two or more coupling elements.

The connecting elements may have a linear shape or a curved shape.

As a material for the stent, it is possible to use stainless steel, tungsten, tantalum, nickel-titanium alloys or the like. In the present invention, the "waved element" means an element that repeatedly meanders like a wave train. In cases where the adjoining annular members are selectively connected at the nearest wave crests and wave troughs by the connecting elements, it means that at least one combination of parts to be connected is selected from among multiple combinations of the nearest wave crests and wave troughs.

According to the present invention, it is possible to expect the following effects: (1) the whole stent is excellent in flexibility to bending because of the annular members that form the tubular wall of the stent being composed of repeated meandering patterns. Thus, it is excellent in trackability to lumens. Further, it is easy to provide the stent with a lateral hole; (2) Each annular member is composed of two waved elements that repeatedly meander in parallel with or substantially parallel with each other and are coupled at the intermediate portions between wave crests and wave troughs by coupling elements. Thus, by increasing the width of the coupling elements, it is possible to improve the radial strength of the stent as well as to improve the flexibility of the stent while meeting the radial strength. Further, since the annular member is composed of two waved elements, it is possible to equalize the width and the thickness of waved elements or struts (In this case, the waved elements are formed into a circular cross-section by electrolytic polishing.) while meeting the requirement for the radial strength, which in turn makes it possible to minimize the curvature deformation (i.e., a phenomenon of outward warpage) of the crest portions (i.e., the top or bottom regions of the wave crests or wave troughs) of the waved element which may occur at the time of expansion of the stent.

The present invention has been outlined as above; a further understanding of the present invention will be given from the following description of some specific embodiments of the present invention. These embodiments are provided only for illustration and are not intended to limit the invention thereto unless otherwise stated.

### Brief Description of Drawings

Fig. 1 is a plan view of a stent according to one embodiment of the present invention;
Fig. 2 is a development of the stent shown in Fig. 1;
Fig. 3 is a plan view illustrating an expanded state of the stent shown in Fig. 1;
Fig. 4 is a partially enlarged view of the stent shown in Fig. 2;
Fig. 5A is a development of a stent according to another embodiment of the present invention;
Fig. 5B is a partially enlarged view of the stent shown in Fig. 5A;
Fig. 6A is a development of a stent according to still another embodiment of the present invention;
Fig. 6B is a partially enlarged view of the stent shown in Fig. 6A;
Fig. 7 is a graph illustrating a comparison of flexibility between the stent of the present invention and that of the prior art;
Fig. 8 is a graph illustrating a comparison of shortening between the stent of the present invention and that of the prior art;
Fig. 9 is graph illustrating a comparison of radial strength between the stent of the present invention and that of the prior art;

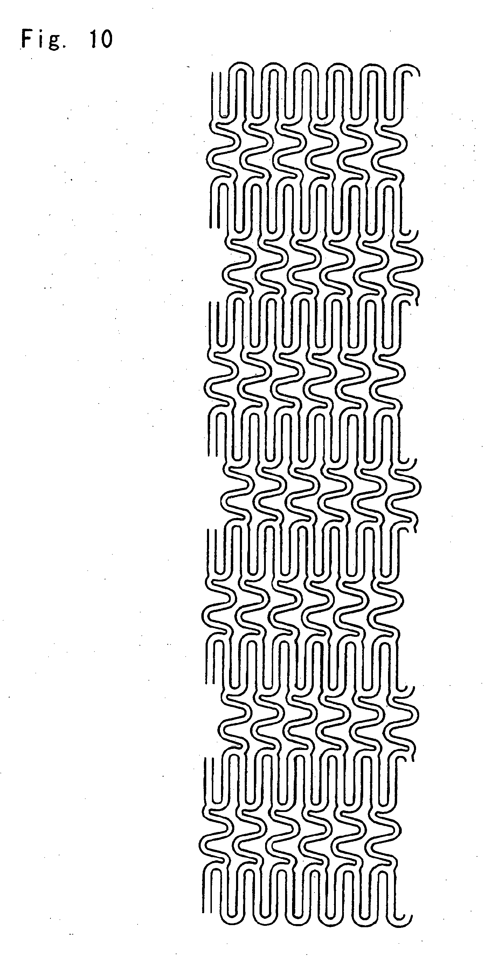

Fig. 10 is a development of a stent of the prior art;

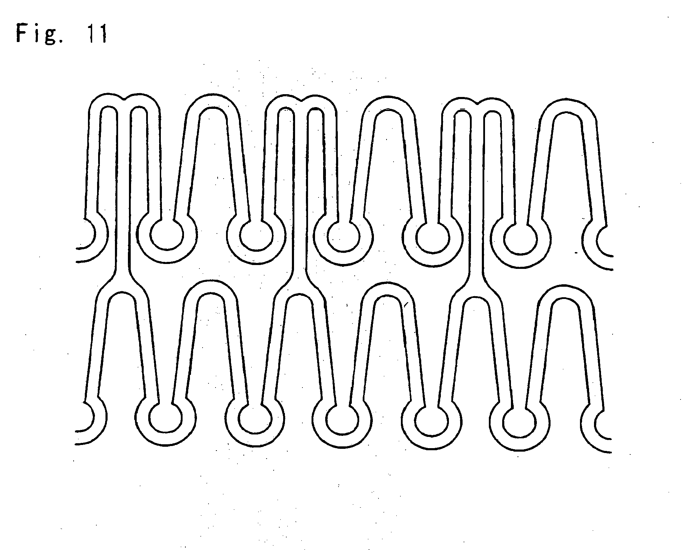

Fig. 11 is a development of a stent of the prior art;

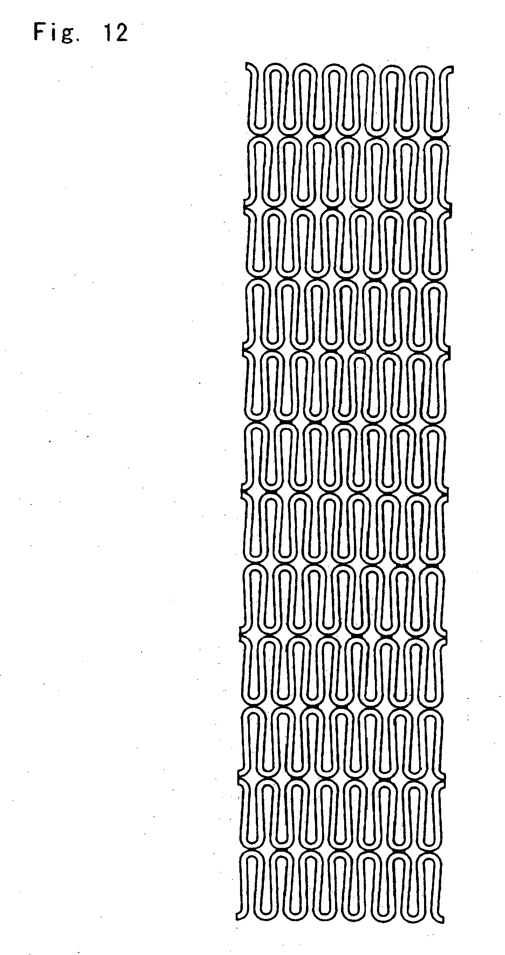

Fig. 12 is a development of a stent of the prior art;

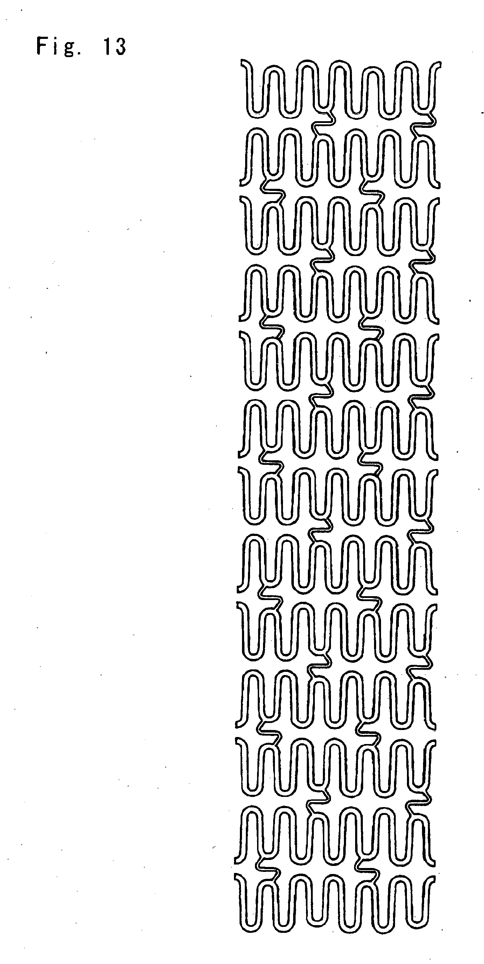

Fig. 13 is a development of a stent of the prior art.

### Best Mode for Carrying Out the Invention

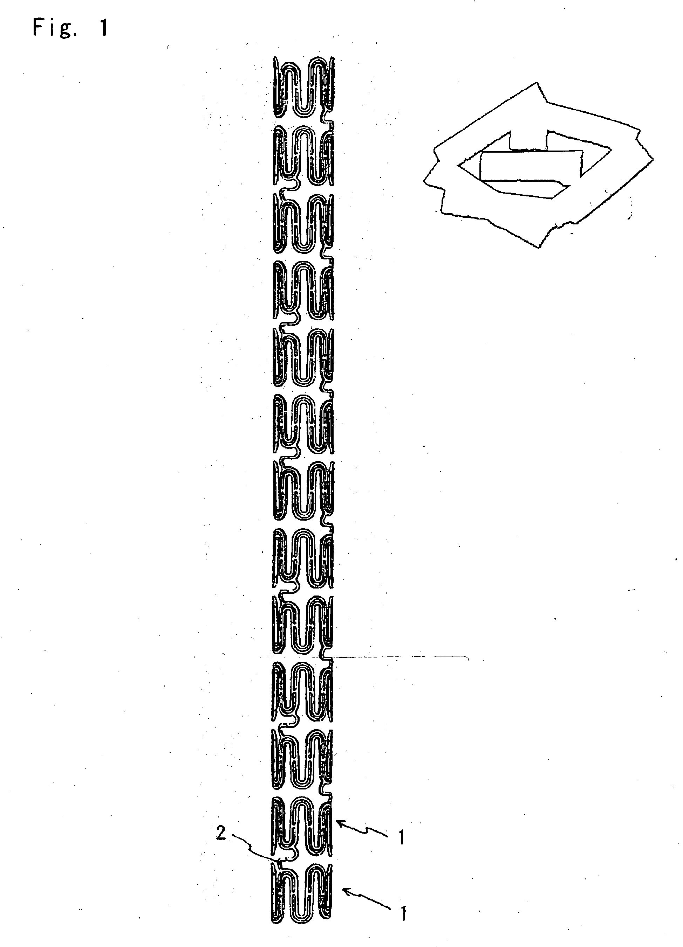

As shown in Figs. 1-6, the stent of the present invention is a tubular member, which is radially expandable and comprises a plurality of annular members 1 arranged in an axial direction of the annular member 1 to keep cavities of the living body open. Adjoining two annular members 1, 1 are respectively connected in the axial direction by one or more connecting elements 2 to form a tubular member.

Each annular member 1 has a proximal end and a distal end and is composed of two waved elements 11, 12 that repeatedly meander in parallel with each other and are coupled at intermediate portions between the proximal end and the distal end of the annular member 1 by coupling elements 13. The adjoining annular members 1, 1 are being selectively connected at the nearest wave crests and wave troughs by the connectinq elements 2.

The waved elements 11, 12 are so constructed that the half-wave sections of the waved elements 11, 12 connected by the connecting elements 2 have an amplitude smaller than that of half-wave sections of the unconnected waved elements, preferably, an amplitude that is 4/5 the amplitude of the sections of the unconnected waved elements. Each annular member 1 is composed of six unit cells (one unit cell is composed of one wave crest and one wave trough). Here, "wave crest" means a section of the waved element that lies above a center line between both ends of each annular member 1, and "wave trough" means a section which lies below the center line (CL) between both ends of each annular member 1.

In another embodiment, the two waved elements 11, 12 are partially uneven and are partially narrowed or widened at the wave crests 111, 121 and the wave troughs 112, 122 of the two waved elements 11, 12 in pair, as compared with parallel portions of the waved elements 11, 12. In this case, the gap between the parallel portions of the two waved elements 11, 12 is set to 50 µm and the connecting elements 2 are in the form of a curved surface shape (e.g., an S-shaped pattern).

### Embodiment 1

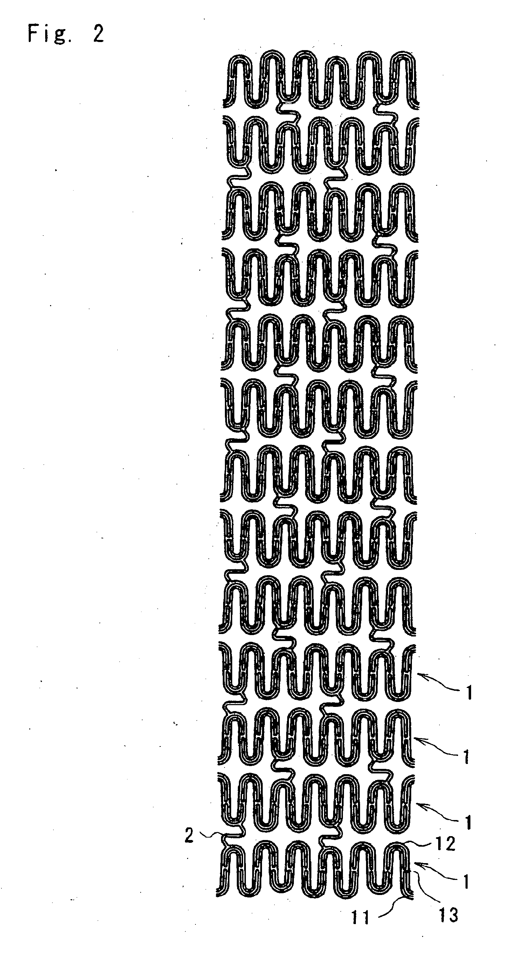

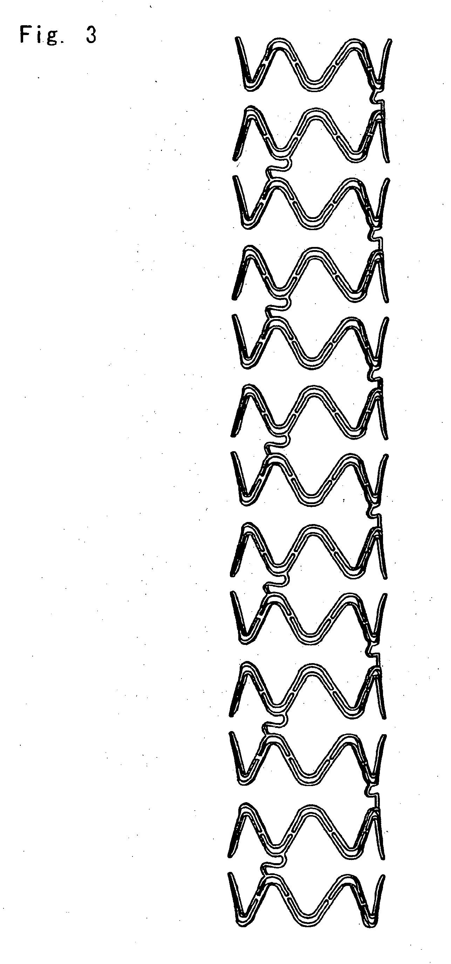

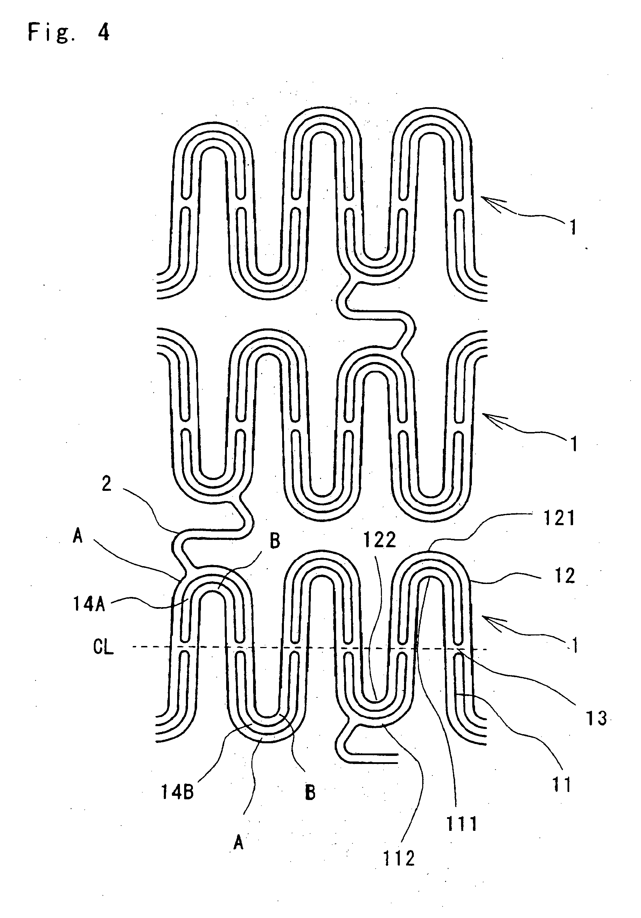

Firstly, a first embodiment of the present invention will be explained below with reference to Figs. 1-4. Fig. 1 is a plan view of a stent according to one embodiment of the present invention; Fig. 2 is a development of the stent shown in Fig. 1; Fig. 3 is a plan view illustrating an expanded state of the stent shown in Fig. 1; and Fig. 4 is a partially enlarged view of Fig. 2.

As shown in Figs. 1-3, a stent of the first embodiment is a radially expandable tubular member comprising thirteen annular members 1 arranged in an axial direction thereof to keep cavities of the living body open, and connecting members 2 arranged between adjoining annular members 1 Lo connect them with two connecting members 2. As shown in detail in Fig. 4, each annular member 1 in an developed state is composed of two waved elements 11, 12 which repeatedly meander in parallel with each other like a wave train and which are coupled by coupling elements 13 at an intermediate position between both ends of the annular element 1. Each annular member 1 is composed of six unit cells (A unit cell is composed of one wave crest and one wave trough).

The two waved elements 11, 12 have the same configuration composed of alternating patterns of a half-wave section A of a long wavelength and a wave section B of a short wavelength. The long wavelength sections A of the waved element 11 have the same amplitude as those of the waved element 12. The same goes for the short wavelength sections B of the waved element 11 and 12. However, the two waved elements 11 and 12 are out of phase by a half-wavelength (1/2) so that the short wavelength section B of one waved element is located within the trough of the long wavelength section A of the other waved element while leaving a gap of 60 µm between them. The two waved elements 11, 12 are coupled by the coupling element 13 at the intermediate portions between both ends of the annular member 1, i.e., between the top of crest 111, 121 and the bottom of a wave trough 122, 112. Thus, the long wavelength section A is separated from the short wavelength section B by a horseshoe or U-shaped gap. In that case, the long wavelength section A is larger than the short wavelength section B by the size corresponding to double the width of a strut of the waved elements 11, 12.

The gap between the long wavelength section A and the short wavelength section B is set to 40 to 70 µm in view of the accuracy of current laser beam machining.

A width and thickness of struts of the annular member 1 (i.e., a width and a thickness of the waved elements 11, 12) are set to 60 µm, respectively. Also, a width of the coupling element 13 is 60 µm. The coupling element 13 is generally so designed as to have a width equal to or slightly greater than the width of the waved elements to improve the radial strength, but there is no limitation on the width of the coupling element 13.

The adjoining two annular members 1, 1 are selectively connected at the nearest crests 121 and troughs 112 thereof by two connecting elements 2. In other words, axisymmetric two pairs of the nearest crest 121 and trough 112 are selected among all the combinations of the nearest crests 121 and troughs 112 which are connectable by the connecting elements 2 (there are 6 pairs). To this end, the waved elements 11, 12 are so constructed that the half-wave sections connected by the connecting elements 2 have an amplitude that is 4/5 the amplitude of the unconnected half-wave sections.

The connecting elements 2 are elements which connect annular members 1 to form a tubular member and which are elements that determine the flexibility of the stent. In case the connecting elements 2 are of the same material, the smaller the thickness and width of the connecting elements 2, the greater the flexibility of the stent. In case the stent is produced by laser processing, the thickness of the connecting elements 2 is the same as that of the waved elements 11, 12, and thus the radial strength of the stent would be substantially determined by the radial strength of the annular members 1. When the material is the same, the radial strength of the annular members 1 is determined by the thickness and width of a skeleton structure (i.e., strut) of the annular members 1. Accordingly, the flexibility of the stent that meets the radial strength is determined by the width of the connecting elements 2. It is preferred for the connecting elements 2 to have a square or circular cross-section to avoid difference in flexibility when bent in different directions.

The above stent is flexible to bending, and thus excellent in trackability to lumens since the annular members that constitute a tubular wall of the stent are composed of repeated meandering patterns. Further, it is easy to form a hole in a lateral side of the stent. The annular members are composed of two parallel waved elements which repeat meandering patterns and which are being coupled at the intermediate portions between the wave crests and wave troughs by the coupling elements, thus greater width of the coupling elements makes it possible to improve the radial strength as well as to improve the flexibility while successfully satisfying the radial strength. In addition, the annular members are composed of two waved elements, thus making it possible to equalize the width and thickness of the struts (In this case, the cross-section of the strut is made into substantially circular shape by electrolytic polishing.) while successfully satisfying the radial strength, which in turn makes it possible to minimize the curvature deformation (a phenomena of outward warpage) of the crest portions of the waved element at the time of expansion of the stent.

### Embodiment 2

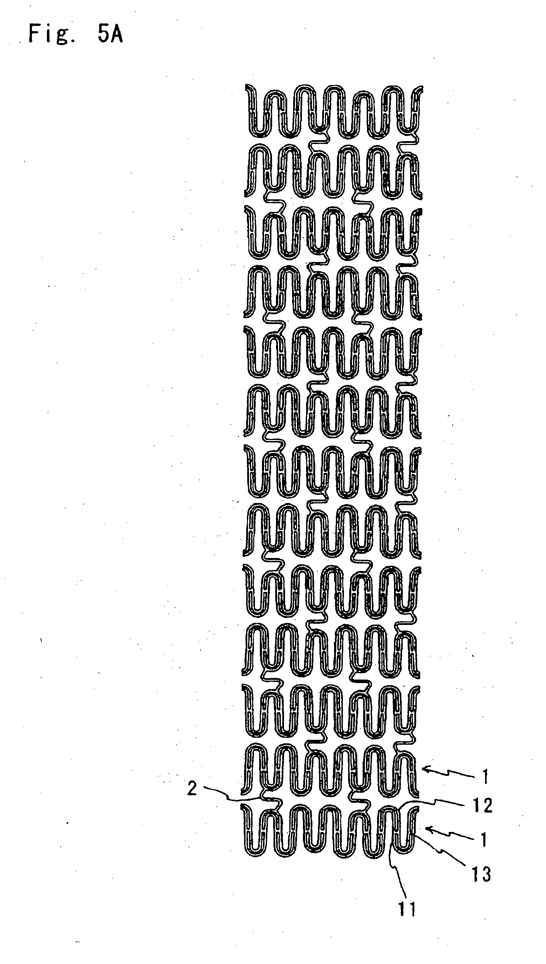

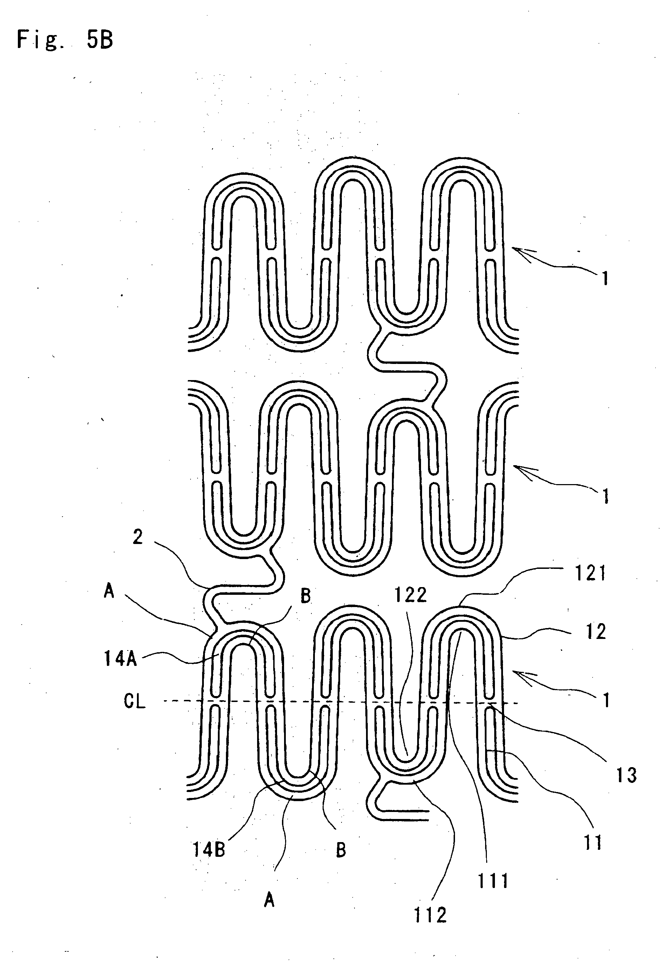

Embodiment 2 of the present invention will be demonstrated below with reference to Fig. 5.

The stent of embodiment 2 has the same configuration as that of the stent of embodiment 1 except for that the gap between the wave crests and the gap between the wave troughs are uneven and are narrowed at the top or bottom portions of the wave crests or wave troughs. As shown in Fig. 5, the gap 14A between the crest portion (111) of the first waved element 11 and the crest portion (121) of the second waved element 12 and the gap 14B between the trough portion (112) of the first waved element 11 and the trough portion (122) of the second waved element 12 are uneven and the gaps 14A and 14B are gradually narrowed from the intermediate portion of the annular member 1 to the top 121 or bottom 112 of the wave crests or wave trough. The gap 14A, 14B at the top 121 or bottom 112 of the wave crests or wave trough is set to 40 µm, which is smaller than the gap (60 µm) in other parts by 20 µm.

The above stent as a whole is flexible to bending and thus excellent in trackability to lumens. Further, it is easy to form a lateral hole in the stent. It is possible to reduce the width and thickness of struts while satisfying the radial strength. Also, it is minimize the curvature deformation (a phenomenon which causes outward warpage) in the crest portions of the waved elements at the time of expansion of the stent by equalizing the width and thickness of the struts. Since there is not so much of a difference between the curvature deformation in the crest portions and trough portions of the first waved element and that in the crest portions and trough portions of the second waved element, it is possible to reduce a difference in pressure acting on the vessel wall, which in turn makes it possible to decrease stress on the vessel wall.

### Embodiment 3

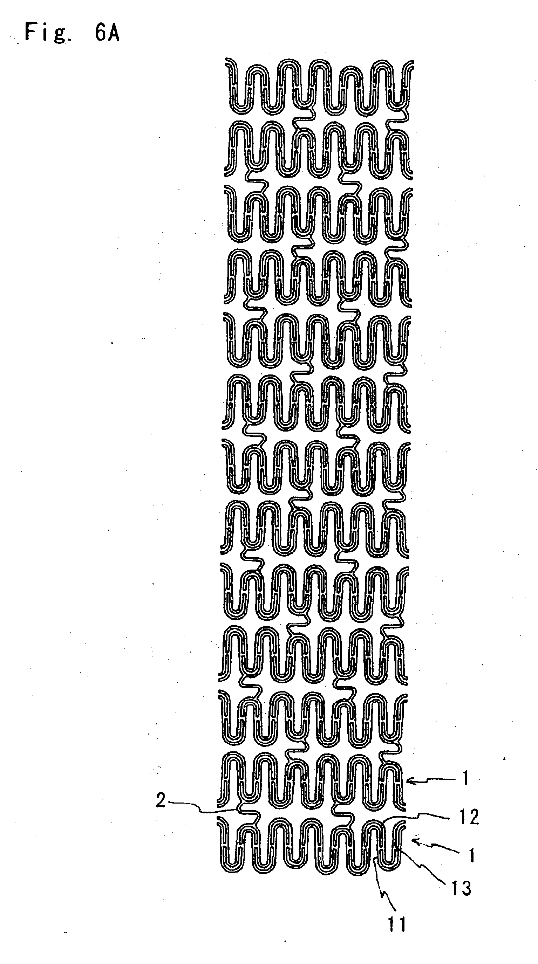

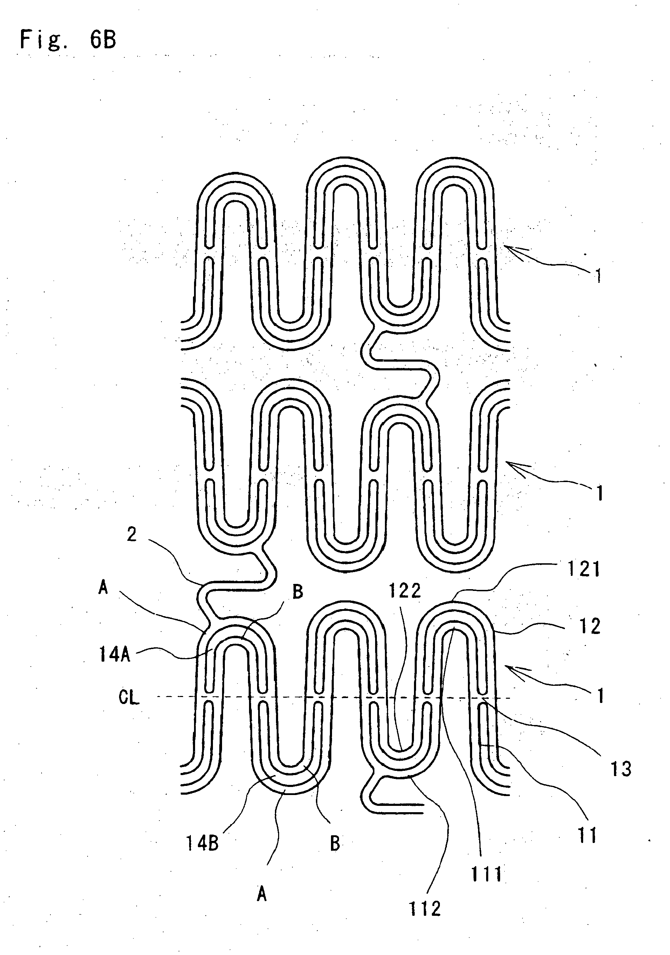

Embodiment 3 of the present invention will be demonstrated below with reference to Fig. 6.

The stent of embodiment 3 has the same configuration as that of the stent of embodiment 1 except for that the gap between the wave crest and the gap between the wave troughs are uneven and are widened at the top or bottom regions of the wave crests or wave troughs as compared with other regions of the wave crests or wave troughs.

As shown in Fig. 6, the gap 14A between the crest portion (111) of the first waved element 11 and the crest portion (121) of the second waved element 12 and the gap 14B between the trough portion (112) of the first waved element 11 and the trough portion (122) of the second waved element 12 are uneven and the gaps 14A and 14B are gradually widened from the intermediate portion of the annular member 1 to the top 121 or bottom 112 of the wave crest or wave trough. The gap 14A, 14B at the top 121 or bottom 112 of the wave crests or wave trough is 80 µm which is greater than the gap (60 µm) in other portions by 20 µm.

The above stent as a whole is flexible to bending and Lhus excellent in trackability to lumens. Further, it is easy to form a lateral hole in the stent. It is possible to reduce the width and thickness of struts while satisfying the radial strength. Also, it is minimize the curvature deformation (a phenomenon which causes outward warpage) in the crest portions of the waved clcments at the time of expansion of the stent by equalizing the width and thickness of the struts. Since there is a great difference between the curvature deformation in the crest portions and trough portions of the first waved element and that in the crest portions and trough portions of the second waved element, it is possible to increase a difference in pressures acting on the vessel wall, which in turn makes it possible to fix the stent to the vessel wall at the outer wave crest portions and outer wave trough portions.

### [Analysis of Flexibility, shortening and radial strength]

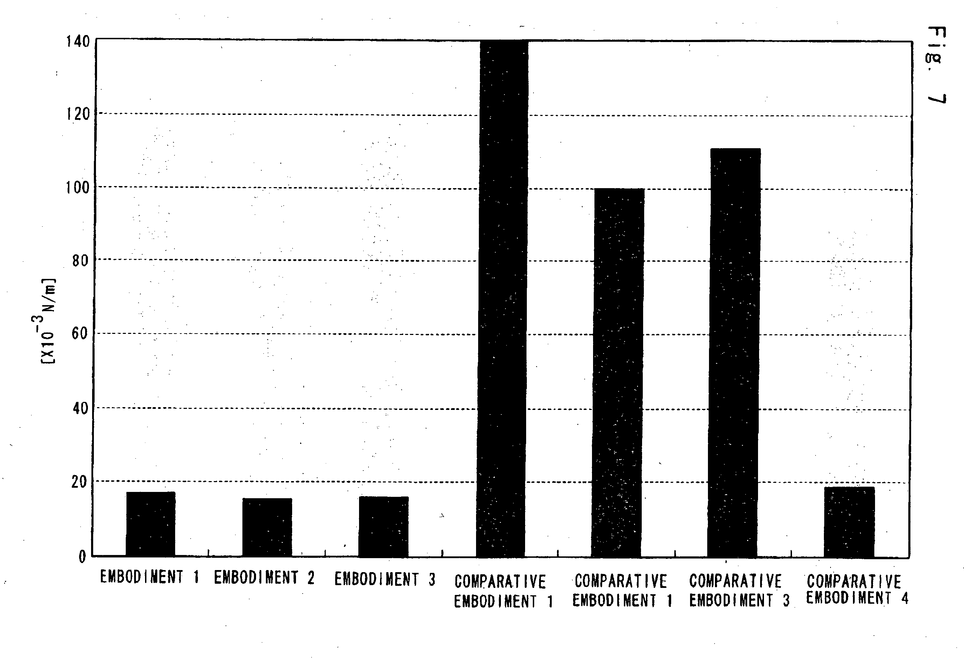

There were conducted simulation analyses of stents made of SUS 316L having a development shown in Table 1. Results achieved in comparisons of data on flexibility (bendability), shortening and radial strength of the stents are shown in Figs. 7-9.

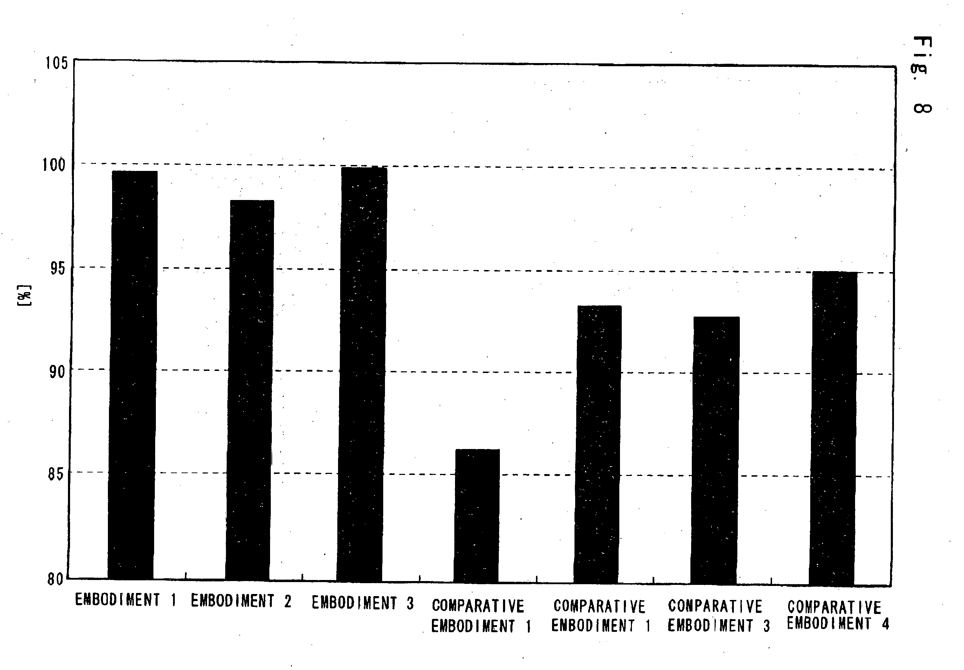

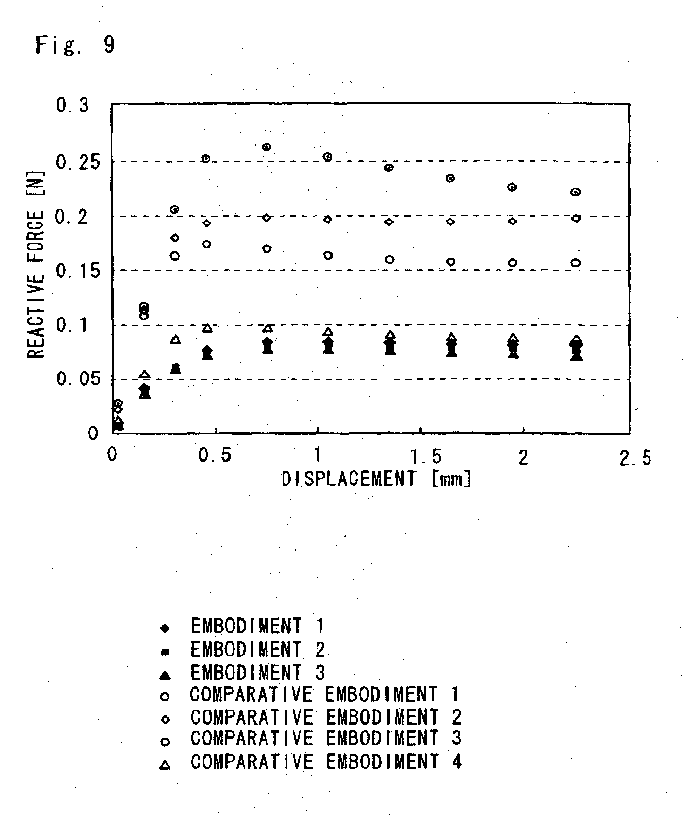

From the results shown in Fig. 7, it is determined that the stent of the present invention is without a doubt superior in flexibility to conventional stents. Further, the results in Fig. 8 show that it is possible to prevent the stent from shortening after expansion by appropriate determination of the amplitude of the crests and troughs to be connected. From the results shown in Fig. 9, it will be understood that the stent of the present invention is inferior in radial strength to the conventional stents, but the comparison with the stent of comparative example 4 shows that the stent of the present invention meets the radial strength.

For the flexibility (bendability), a displacement magnitude (mm) of the stent was determined by fixing the stent at one end and applying a load (Newton) to the other end of the stent.

The shortening was determined by measuring a change in length before and after expanding the stent to a diameter of 3.0 mm. The graph shows rates of length change of the stents (length before expansion/length after expansion).

The radial strength was determined by measuring the change of diameter (mm) of the stent when applying compressing force (Newton) to the stent.

**TABLE 1**

| | Remarks |
|---|---|
| Embodiment 1 | Fig. 2(amplitude of sections to be connected/amplitude of sections to be un connected: 4/5, shape of the connecting element: S-shaped, phase sift: 1/2 wavelength, two waved elements are parallel) |
| Embodiment 2 | Fig. 5(The gaps between the tops of the crests and between the bottoms of the crests are narrowed as compared with that in Fig. 2.) |
| Embodiment 3 | Fig. 6(The gaps between the tops of the crests and between the bottoms of the crests are widened as compared with that in Fig. 2.) |
| Comparative embodiment 1 | Fig. 10(blood vessel-holding portions of a waved pattern and waved joint elements) |
| Comparative embodiment 2 | Fig. 11(blood vessel-holding portions of a waved pattern, jointed between crests and troughs of the waved patterns.) |
| Comparative embodiment 3 | Fig. 12(blood vessel-holding portions of a waved pattern, jointed between crests of the waved patterns) |
| Comparative embodiment 4 | Fig. 13 (annular members has a shape similar to that of annular members in embodiment 1 but is being composed of a single waved element. The waved element is so designed that a width of the waved element is two times as large as that of the waved element in embodiment 1 and that a center line of the waved element is matched with a line passing through the center of the two waved elements in Embodiment 1.) |

## Claims

1. A flexible stent with excellent trackability and expandability, comprising a plurality of radially expandable annular members (1) arranged in an axial direction thereof, and connecting elements (2) for connecting adjoining annular members (1) in the axial direction of the stent, wherein:
each of said annular members (1) has a proximal end and a distal end and comprises two waved elements (11, 12) that repeatedly meander in parallel with or substantially parallel with each other to form a parallel or substantially parallel meandering pattern with wave crests (111, 121) and wave troughs (112, 122), and coupling elements (13) for coupling the two waved elements (11, 12) together;
said two waved elements (11, 12) are separated from one another by a gap (14A, 14B) of 40 to 70 µm along the entire length of said meandering patterns; and
said adjoining armular members (1, 1) are selectively connected at the nearest wave crests (121) and wave troughs (112) of said waved elements (11, 12) of the adjoining annular members (1, 1) by one or more of said connecting elements (2), **characterised in that** said two waved elements (11, 12) are coupled by said coupling elements (13) at each intermediate portion between the proximal end and the distal end of said annular member (1).

2. The stent according to claim 1, wherein said two waved elements (11, 12) are in parallel with each other.

3. The stent according to claim 1, wherein the gap (14A, 14B) between said two waved elements (11, 12) is partially uneven and both narrows at the top portions of the wavecrests (111, 121) and at the bottom portions of the wave troughs (112, 122) of the two waved elements (11, 12).

4. The stent according to claim 1, wherein the gap (14A, 14B) between said two waved elements (11, 12) is partially uneven and both widens at the top portions of the wave crests (111, 121) and at the bottom portions of the wave troughs (112, 122) of the two waved elements (11, 12).

5. The stent according to any one of claims 2 to 4, wherein the gap (14A, 14B) is in the range of 40 to 70 µm at parallel parts of said two waved elements (11, 12).

6. The stent according to claim 1, wherein the connecting element (2) is formed in a linear shape.

7. The stent according to claim 1, wherein the connecting element (2) is formed in a curved shape.

8. The stent according to claim 1, wherein each of said two waved elements (11,12) are composed of alternating patterns of a half-wave section with a long wavelength (A) and a half-wave section with a short wavelength (B) and are arranged so that the half-wave section with a short wavelength (B) of one of the two waved elements (11,12) is located in the half-wave section with a long wavelength (A) of the other waved element (11, 12) is located in the half-wave section with a long wavelength (A) of the other waved element (11, 12) while leaving a gap (14A, 14B) between them.

9. The stent according to claim 8, wherein the half-wave section with a long wavelength (A) of one of the two waved elements (11, 12) is separated from the half-wave sections with a short wavelength (B) of the other waved element (11, 12) by a U-shaped gap (14A, 14B).

10. The stent according to claim 8 or claim 9, wherein said half-wave sections with a long wavelength (A) connected by the axial connecting elements (2) have an amplitude that is 4/5 the amplitude of the unconnected half-wave sections (A).

11. The stent according to claim 1, wherein the coupling elements (13) have a width equal to or slightly greater than the width of the waved elements (11, 12).

12. The stent according to claim 1 or claim 10, wherein said adjoining wave crests (121) and wave troughs (112) that are connected by said axial connecting elements (2) have an amplitude smaller than that of the wave crests (121) and wave troughs (112) that are not connected by the connecting elements (2).

## Patentansprüche

1. Flexibler Stent mit einer ausgezeichneten Führbarkeit und Dehnbarkeit, mit mehreren radial dehnbaren ringförmigen Elementen (1), die in einer axialen Richtung des Stent angeordnet sind, und Verbindungselementen (2) zum Verbinden benachbarter ringförmiger Elemente (1) in der axialen Richtung des Stent, wobei
jedes der ringförmigen Elemente (1) ein proximales Ende und ein distales Ende aufweist und zwei wellenförmige Elemente (11, 12), die sich parallel oder im Wesentlichen Parallel zueinander wiederholt schlängeln, um ein paralleles oder im Wesentlichen paralleles sich schlängelndes Muster mit Wellenbergen (111, 121) und Wellentälern (112, 122) zu bilden, und Verbindungselemente (13) zum Verbinden der wellenförmigen Elemente (11, 12) miteinander;
wobei die beiden wellenförmigen Elemente (11, 12) über einen Zwischenraum (14A, 14B) von 40 bis 70 µm entlang der gesamten Länge der sich schlängelnden Muster voneinander getrennt sind; und
wobei die einander benachbarten ringförmigen Elemente (1, 1) an den nähesten Wellenbergen (121) und Wellentälern (112) der wellenförmigen Elemente (11, 12) der benachbarten ringförmigen Elemente durch ein oder mehrere der Verbindungselemente (2) selektiv verbunden sind;
**dadurch gekennzeichnet, dass**
die beiden wellenförmigen Elemente (11, 12) durch die Verbindungselemente (13) an jedem Zwischenabschnitt zwischen dem proximalen Ende und dem distalen Ende des ringförmigen Elements (1) verbunden sind.

2. Stent nach Anspruch 1, wobei die beiden wellenförmigen Elemente (11, 12) parallel zueinander angeordnet sind.

3. Stent nach Anspruch 1, wobei der Zwischenraum (14A, 14B) zwischen den beiden wellenförmigen Elementen (11, 12) teilweise ungleichmäßig ist und sich sowohl an den oberen Abschnitten der Wellenberge (111, 121) als auch an den unteren Abschnitten der Wellentäler (112, 122) der beiden wellenförmigen Elemente (11, 12) verschmälert.

4. Stent nach Anspruch 1, wobei der Zwischenraum (14A, 14B) zwischen den beiden wellenförmigen Elementen (11, 12) teilweise ungleichmäßig ist und sich sowohl an den oberen Abschnitten der Wellenberge (111, 121) als auch an den unteren Abschnitten der Wellentäler (112, 122) der beiden wellenförmigen Elemente (11, 12) verbreitert.

5. Stent nach einem der Ansprüche 2 bis 4, wobei der Zwischenraum (14A, 14B) an parallelen Abschnitten der beiden wellenförmigen Elemente (11, 12) im Bereich von 40 bis 70 µm liegt.

6. Stent nach Anspruch 1, wobei das Verbindungselement (2) in einer geraden Form ausgebildet ist.

7. Stent nach Anspruch 1, wobei das Verbindungselement (2) in einer gekrümmten Form ausgebildet ist.

8. Stent nach Anspruch 1, wobei jedes der beiden wellenförmigen Elemente (11, 12) aus abwechselnden Mustern aus einem Halbwellenabschnitt mit einer langen Wellenlänge (A) und einem Halbwellenabschnitt mit einer kurzen Wellenlänge (B) besteht, die derart angeordnet sind, dass der Halbwellenabschnitt mit einer kurzen Wellenlänge (B) eines der beiden wellenförmigen Elemente (11, 12) im Halbwellenabschnitt mit einer langen Wellenlänge (A) des anderen wellenförmigen Elements (11, 12) angeordnet ist, während dazwischen ein Zwischenraum (14A, 14B) belassen wird.

9. Stent nach Anspruch 8, wobei der Halbwellenabschnitt mit einer langen Wellenlänge (A) eines der beiden wellenförmigen Elemente (11, 12) von den Halbwellenabschnitten mit einer kurzen Wellenlänge (B) des anderen wellenförmigen Elements (11, 12) durch einen U-förmigen Zwischenraum (14A, 14B) getrennt ist.

10. Stent nach Anspruch 8 oder 9, wobei die durch die axialen Verbindungselemente (2) verbundenen Halbwellenabschnitte mit einer langen Wellenlänge (A) eine Amplitude haben, die 4/5 der Amplitude der nicht verbundenen Halbwellenabschnitte (A) beträgt.

11. Stent nach Anspruch 1, wobei die Verbindungselemente (13) eine Breite haben, die der Breite der wellenförmigen Elemente (11, 12) gleicht oder etwas größer als diese ist.

12. Stent nach Anspruch 1 oder 10, wobei die benachbarten Wellenberge (121) und Wellentäler (112), die durch die axialen Verbindungselemente (2) verbunden sind, eine Amplitude haben, die kleiner ist als diejenige der Wellenberge (121) und Wellentäler (112), die nicht durch die Verbindungselemente (2) verbunden sind.

## Revendications

1. Implant vasculaire souple présentant d'excellentes propriétés de guidage et d'expansion, comprenant une pluralité d'éléments annulaires pouvant être expansés radialement (1) agencés dans la direction axiale de celui-ci, et des éléments de liaison (2) destinés à relier des éléments annulaires adjacents (1) dans la direction axiale de l'implant vasculaire, dans lequel :
chacun desdits éléments annulaires (1) présente une extrémité proximale et une extrémité distale et comprend deux éléments ondulés (11, 12) qui forment des méandres répétitifs en parallèle ou sensiblement en parallèle l'un par rapport à l'autre afin de former un profil en méandres parallèles ou sensiblement parallèles avec des crêtes d'onde (111, 121) et des creux d'onde (112, 122), et des éléments de couplage (13) destinés à coupler les deux éléments ondulés (11, 12) entre eux ;
lesdits deux éléments ondulés (11, 12) sont séparés l'un de l'autre par un intervalle (14A, 14B) de 40 à 70 µm suivant la totalité de la longueur desdits profils en méandres ; et
lesdits éléments annulaires adjacents (1, 1) sont reliés sélectivement au niveau des crêtes d'onde (121) et creux d'onde (112) les plus proches desdits éléments ondulés (11, 12) des éléments annulaires adjacents (1, 1) par un ou plusieurs desdits éléments de liaison (2), **caractérisé en ce que** lesdits deux éléments ondulés (11, 12) sont couplés par lesdits éléments de couplage (13) au niveau de chaque partie intermédiaire entre l'extrémité proximale et l'extrémité distale dudit élément annulaire (1).

2. Implant vasculaire selon la revendication 1, dans lequel lesdits deux éléments ondulés (11, 12) sont parallèles l'un à l'autre.

3. Implant vasculaire selon la revendication 1, dans lequel l'intervalle (14A, 14B) entre lesdits deux éléments ondulés (11, 12) est partiellement irrégulier et se rétrécit à la fois au niveau des parties supérieures des crêtes d'onde (111, 121) et au niveau des parties inférieures des creux d'onde (112, 122) des deux éléments ondulés (11,12).

4. Implant vasculaire selon la revendication 1, dans lequel l'intervalle (14A, 14B) entre lesdits deux éléments ondulés (11, 12) est partiellement irrégulier et s'élargit à la fois au niveau des parties supérieures des crêtes d'onde (111, 121) et au niveau des parties inférieures des creux d'onde (112, 122) des deux éléments ondulés (11, 12).

5. Implant vasculaire selon l'une quelconque des revendications 2 à 4, dans lequel l'intervalle (14A, 14B) est compris dans la plage de 40 à 70 µm au niveau des parties parallèles desdits deux éléments ondulés (11, 12).

6. Implant vasculaire selon la revendication 1, dans lequel l'élément de liaison (2) est réalisé suivant une forme linéaire.

7. Implant vasculaire selon la revendication 1, dans lequel l'élément de liaison (2) est réalisé suivant une forme courbe.

8. Implant vasculaire selon la revendication 1, dans lequel chacun desdits deux éléments ondulés (11, 12) est composé de profils alternés d'une section en demi-onde avec une longueur d'onde longue (A) et d'une section en demi-onde avec une longueur d'onde courte (B) et est agencé de telle sorte que la section en demi-onde avec une longueur d'onde courte (B) de l'un des deux éléments ondulés (11, 12) est située sur la section en demi-onde avec une longueur d'onde longue (A) de l'autre élément ondulé (11, 12), (est située sur la section en demi-onde avec une longueur d'onde longue (A) de l'autre l'élément ondulé (11, 12)), tout en laissant un jeu (14A, 14B) entre eux.

9. Implant vasculaire selon la revendication 8, dans lequel la section en demi-onde avec une longueur d'onde longue (A) de l'un des deux éléments ondulés (11, 12) est séparée des sections en demi-onde avec une longueur d'onde courte (B) de l'autre élément ondulé (11, 12) par un intervalle en forme de U (14A, 14B).

10. Implant vasculaire selon la revendication 8 ou 9, dans lequel lesdites sections en demi-onde avec une longueur d'onde longue (A) reliées par les éléments de liaison axiaux (2) présentent une amplitude qui est de 4/5 de l'amplitude des sections en demi-onde (A) non raccordées.

11. Implant vasculaire selon la revendication 1, dans lequel les éléments de couplage (13) présentent une largeur égale ou légèrement supérieure à la largeur des éléments ondulés (11, 12).

12. Implant vasculaire selon la revendication 1 ou 10, dans lequel lesdits crêtes d'onde (121) et creux d'onde (112) adjacents qui sont reliés par lesdits éléments de liaison axiaux (2) présentent une amplitude inférieure à ceux des crêtes d'onde (121) et creux d'onde (112) qui ne sont pas reliés par les éléments de liaison (2).
